# EUROPÄISCHE PATENTSCHRIFT

(11) **EP 2 764 358 B1**
(45) Veröffentlichungstag und Bekanntmachung des Hinweises auf die Patenterteilung: **12.07.2017**
(21) Anmeldenummer: 12781283.2
(22) Anmeldetag: 02.10.2012
(51) Int. Cl.: G01N 33/24

(54) **SENSORMODUL**
SENSOR MODULE
MODULE DE CAPTEUR

(30) Priorität: 04.10.2011 DE 102011083989
(43) Veröffentlichungstag der Anmeldung: 13.08.2014
(73) Patentinhaber: Schunk Wien Gesellschaft m.b.H., 1230 Wien (AT); Texplor Austria GmbH, 1220 Wien (AT)
(72) Erfinder: DENNER, Gerhard, A-1230 Wien (AT); PLACH, Werner, A-3032 Eichgraben (AT)
(74) Vertreter: advotec.
(86) Internationale Anmeldenummer: PCT/EP2012/069493
(87) Internationale Veröffentlichungsnummer: WO 2013/050387

(56) Entgegenhaltungen:
- EP-A2- 0 354 733
- WO-A2-2009/157755
- WO-A2-2009/157755
- DE-A1- 2 822 769
- DE-T2- 69 014 985

## Beschreibung

Die Erfindung betrifft ein Sensormodul zur Messung einer elektrischen Größe mit zumindest einer Elektrode nach dem Oberbegriff des Anspruchs 1.

Sensormodule der eingangs genannten Art können zur Bestimmung lokaler elektrischer Größen, wie beispielsweise des Widerstands, der Stromstärke oder der Spannung, in einer Festkörperumgebung, beispielsweise im Erdreich, verwendet werden. Da sich bekanntermaßen elektrische Größen, wie beispielsweise der elektrische Widerstand, im Erdreich in Abhängigkeit von der Zusammensetzung des Erdreichs, insbesondere der Erdreichformation und dem Flüssigkeitsgehalt bzw. der Flüssigkeitsverteilung im Erdreich, ändern, werden derartige Sensormodule beispielsweise eingesetzt, um Leckagen in Abdichtungen von Deponien und Lagerstellen zu detektieren. Je nach Art der Deponielagerstoffe sind dabei die Sensormodule einer teilweise erheblich aggressiven Umgebung ausgesetzt, sodass es neben der guten elektrischen Leitfähigkeit der Kontaktoberflächen der Sensormodule für die Funktionssicherheit der Sensormodule wesentlich ist, dass sie sich als chemisch beständig erweisen. Darüber hinaus ist es natürlich für die Funktionssicherheit von Sensormodulen der eingangs genannten Art ganz wesentlich, dass sie eine ausreichende mechanische Stabilität aufweisen, um den im Erdreich auftretenden mechanischen Belastungen standzuhalten.

Aus der DE 28 22 769 A1 ist ein Sensormodul mit einer Elektrode bekannt, die ein zwischen zwei Kontaktplatten aus einem elektrisch leitenden Kohlenstoffmaterial angeordnetes Sensorelement aufweist.

Die WO 2009/157755 A2 zeigt ein Sensormodul mit einem im Inneren eines Gehäuses angeordneten Sensorelement, das mit einer perforierten Gehäusewandung, auf deren Innenfläche eine poröse Polymerbeschichtung angeordnet ist, abgedeckt ist.

Der vorliegenden Erfindung liegt die Aufgabe zugrunde, ein Sensormodul vorzuschlagen, das neben einer guten elektrischen Kontaktfähigkeit, also einem geringen Kontaktwiderstand, eine große chemische Beständigkeit und darüber hinaus eine große mechanische Stabilität aufweist. Diese Anforderungen betreffen natürlich im besonderen Maße die in dem Sensormodul angeordneten Elektroden, die in unmittelbarem Kontakt zur Umgebung stehen und damit den Umgebungseinflüssen entsprechend unmittelbar ausgesetzt sind.

Zur Lösung dieser Aufgabe weist das erfindungsgemäße Sensormodul die Merkmale des Anspruchs 1 auf.

Erfindungsgemäß weist die Elektrode des Sensormoduls ein zwischen einer inneren und einer äußeren Kontaktplatte aus einem elektrisch leitenden Kohlenstoffmaterial angeordnetes Sensorelement auf, das mit dem Anschlusskabel verbunden ist, wobei eine äußere Kontaktplatte der Elektrode in einer Außenfläche des Gehäuses angeordnet ist. Durch die Anordnung des Sensorelements, das beispielsweise aus einem mäanderförmig ausgebildetes Sensordraht bestehen kann, zwischen den beiden Kontaktplatten aus einem Kohlenstoffmaterial, ist eine Unterbringung des Sensorelements ermöglicht, die neben einer guten elektrischen Leitfähigkeit einen mechanischen Schutz des empfindlichen Sensorelements bietet, und darüber hinaus die für die Funktionsfähigkeit des Sensorelements auch in einer chemisch aggressiven Umgebung notwendige chemische Beständigkeit aufweist.

Vorzugsweise kann das Sensorelement aus nicht korrodierendem Material bzw. mit einer nicht korrodierenden Beschichtung versehenem Material gebildet sein. Beispielsweise kann das Sensorelement eine Goldbeschichtung aufweisen.

Insgesamt ist somit durch die sandwichartige Aufnahme des Sensorelements zwischen den Kontaktplatten aus Kohlenstoff eine schützende Abschirmung des Sensorelements gegenüber mechanischer und chemischer Beanspruchung sowie gleichzeitig auch eine sichere elektrische Kontaktierung zwischen dem den Kontaktplatten zugewandten, beispielsweise durch Erdreich gebildeten Umgebungsbereich und dem zwischen den Kontaktplatten angeordneten Sensorelement möglich.

Zur Verwendung als Kontaktplatte eignet sich in besonderem Maße eine elektrisch leitende Platte, die als gas- und flüssigkeitsdichte Platte in einem Heißpressverfahren aus einem Kohlenstoffmaterial, das eine Mischung aus einem Duroplast und Kohlenstoffpartikeln aufweist, hergestellt ist, und die nach dem Formungsvorgang carbonisiert und zur Erzielung der Gas- und Flüssigkeitsdichtheit imprägniert worden ist. Die Herstellung einer derartigen Platte, die sich durch eine hohe Formstabilität auszeichnet, ist in der WO 2000/016424 beschrieben, deren Offenbarungsgehalt hiermit aufgenommen wird.

Vorteilhaft kann das Sensorelement bereits bei der Formung einer derartigen Kontaktplatte in dem Kohlenstoffmaterial der Kontaktplatte angeordnet werden, so dass bei der fertiggestellten Kontaktplatte das Sensorelement in der Kontaktplatte eingebettet aufgenommen ist.

Um eine definierte Anordnung des Sensorelements zwischen den Kontaktplatten zu ermöglichen, ist es vorteilhaft, wenn zur Anordnung des Sensorelements zwischen den Kontaktplatten eine in einer Verbindungsebene der Kontaktplatten ausgebildete Aufnahmekammer ausgebildet ist.

Um eine möglichst sichere Kontaktierung zwischen dem Sensorelement und den Kontaktplatten zu ermöglichen, kann die Aufnahmekammer zur Einbettung des Sensorelements mit einer elektrisch leitfähigen Füllmasse verfüllt sein, wobei sich hierzu insbesondere Kohlenstoff, beispielsweise in einer Ausbildung als sogenannte "Stampfmasse" eignet, die nach Anordnung des Sensorelements in der Aufnahmekammer in die Aufnahmekammer eingefüllt und in dieser verdichtet werden kann, sodass ein besonders geringer Kontaktwiderstand zwischen dem Sensorelement und den Kontaktplatten aus Kohlenstoff realisierbar ist.

Besonders vorteilhaft ist es auch, wenn die Aufnahmekammer durch eine der Struktur des Sensorelements angepasste Ausnehmung in zumindest einer Kontaktplattenoberfläche ausgebildet ist, wobei die Aufnahmekammer vorzugsweise durch eine Einfräsung in der Kontaktplattenoberfläche ausgebildet ist.

Eine besonders sichere und geschützte Anordnung der Elektrode wird möglich, wenn die Elektrode auf einer Seite einer Trägerplatte des Gehäuses angeordnet ist, derart, dass die Kontaktplatten der Elektrode in einer Elektrodenaufnahme in einer Trägerplattenoberfläche angeordnet sind.

Wenn die Elektrodenaufnahme mit einem an einem Aufnahmeboden der Elektrodenaufnahme ausgebildeten Auflagerand versehen ist, derart, dass die innere Kontaktplatte auf dem Aufnahmeboden und die äußere Kontaktplatte auf dem Auflagerand angeordnet ist, ist ein besonders sicherer Verbund zwischen der Elektrode und dem Gehäuse bzw. der Trägerplatte möglich.

Zur Fixierung der Elektrode bzw. der Kontaktplatten an der Trägerplatte sowie gleichzeitig zur Abdichtung der Elektrode gegenüber der Umgebung ist es vorteilhaft, wenn ein zwischen der äußeren Kontaktplatte und einem Öffnungsrand der Elektrodenaufnahme in der Trägerplattenoberfläche gebildeter Randspalt mit einem nicht leitenden, aushärtenden Kunststoffmaterial verfüllt ist.

Um eine adhäsive Haftung zwischen der Elektrode und der Trägerplatte noch zu verstärken, kann auch der Boden der Elektrodenaufnahme sowie gegebenenfalls zusätzlich auch der Auflagerand mit einer Beschichtung aus einem nicht leitendem, aushärtendem Kunststoffmaterial versehen sein, sodass eine größtmögliche Oberfläche der Elektrode adhäsiv mit der Trägerplatte verbunden ist.

Insbesondere zur Durchführung der Messung einer elektrischen Potenzialdifferenz mittels nur eines Sensormoduls ist es vorteilhaft, wenn das Sensormodul zwei elektrisch voneinander isolierte Elektroden in dem Gehäuse aufweist, die über ein Anschlusskabel an eine elektrische Messeinrichtung anschließbar sind, wobei die Sensorelemente der Elektroden jeweils mit dem Anschlusskabel verbunden sind, und jeweils eine äußere Kontaktplatte jeder Elektrode in einer Außenfläche des Gehäuses angeordnet ist. Hierdurch kann die Verwendung zweier separater Sensormodule durch die Verwendung lediglich eines mit zwei Elektroden versehenen Sensormoduls ersetzt werden.

Bei einer besonders vorteilhaften Relativanordnung der zwei Elektroden befinden sich die Elektroden auf zwei einander gegenüberliegenden Seiten der Trägerplatte des Gehäuses, derart, dass jeweils die Kontaktplatten einer Elektrode in einer Elektrodenaufnahme in einer Trägerplattenoberfläche angeordnet sind. Somit kann beispielsweise bei Integration der Trägerplatte in ein flächenhaft abdichtendes Medium oder Dichtelement dieses Dichtelement dauerhaft überwacht werden.

Besonders vorteilhaft für eine gegen Einwirkung äußerer Einflüsse abgedichteten Aufnahme der Elektroden in das Gehäuse des Sensormoduls ist es, wenn die Trägerplatte benachbart den einander gegenüberliegend angeordneten Elektrodenaufnahmen in der Trägerplattenoberfläche eine Kabelaufnahme zur Aufnahme einer zwischen einem Anschlussende des Anschlusskabels und Sensordrahtenden der Sensorelemente hergestellte Kabelverbindung aufweist, wobei zur Herstellung der Verbindung des Anschlusskabels mit den Sensordrahtenden die Elektrodenaufnahmen über Kabeldurchführungen mit der Kabelaufnahme verbunden sind.

Zur Fixierung des Anschlusskabels in der Kabelaufnahme bzw. am Gehäuse des Sensormoduls erweist es sich vorteilhaft, wenn die Kabelaufnahme mit einem nicht leitenden, aushärtenden Kunststoffmaterial verfüllt ist.

Insbesondere für den Fall, dass zur Verfüllung der Kabelaufnahme sowie gegebenenfalls zur Verfüllung des zwischen den äußeren Kontaktplatten der Elektroden und der Trägerplatte ausgebildeten Randspalten ein vom Material der Trägerplatte abweichendes Material verwendet werden sollte, also beispielsweise in dem Fall, dass für die Trägerplatte ein Polyethylen, insbesondere ein hochdichtes Polyethylen (HDPE) verwendet wird und für das Füllmaterial ein Polyurethan (PU)-Werkstoff, ist es vorteilhaft, wenn die Trägerplatte zumindest auf der die Kabelaufnahme aufweisenden Oberfläche mit einer Beschichtung versehen ist, also etwa mit einer Kunststofffolie versehen ist, die in dem Fall, dass für die Trägerplatte ein Polyethylen als Werkstoff gewählt wird, vorteilhafterweise als Polyethylenfolie ausgebildet ist. Somit kann die Trägerplatte beispielsweise in Foliensysteme als permanente Monitoring-Sensorik eingeschweißt werden.

Das erfindungsgemäße Sensormodul eignet sich - insbesondere aufgrund des eingesetzten Kohlenstoffmaterials - zur Verwendung in der Medizintechnik. Dabei kommt bei einem entsprechend ausgebildeten Sensormodul mit nach außen abgedichtet in der Elektrodenaufnahme der Trägerplatte aufgenommener Elektrode bzw. Elektroden die Verwendung in Körperflüssigkeiten in Betracht.

Eine vorteilhafte Ausführungsform des erfindungsgemäßen Sensormoduls wird nachfolgend anhand der Zeichnungen näher erläutert.

Es zeigen:
- **Fig. 1**: Eine Schnittdarstellung eines Sensormoduls mit zwei Elektroden, die mit äußeren Kontaktplatten in der Oberfläche des Sensormoduls angeordnet sind;
- **Fig. 2**: eine Draufsicht auf das in **Fig. 1** dargestellte Sensormodul;
- **Fig. 3**: eine Draufsicht auf eine Elektrode des in **Fig. 1** dargestellten Sensormoduls;
- **Fig. 4**: eine Draufsicht auf eine Trägerplatte des in **Fig. 1** dargestellten Sensormoduls;
- **Fig. 5**: eine Schnittdarstellung der in **Fig. 4** dargestellten Trägerplatte gemäß Schnittlinienverlauf V - V in **Fig. 4**;
- **Fig. 6**: eine Schnittdarstellung der in **Fig. 4** dargestellten Trägerplatte gemäß Schnittlinienverlauf VI - VI in **Fig. 4**;
- **Fig. 7**: eine Schnittdarstellung der in **Fig. 4** dargestellten Trägerplatte gemäß Schnittlinienverlauf VII - VII in **Fig. 4**.

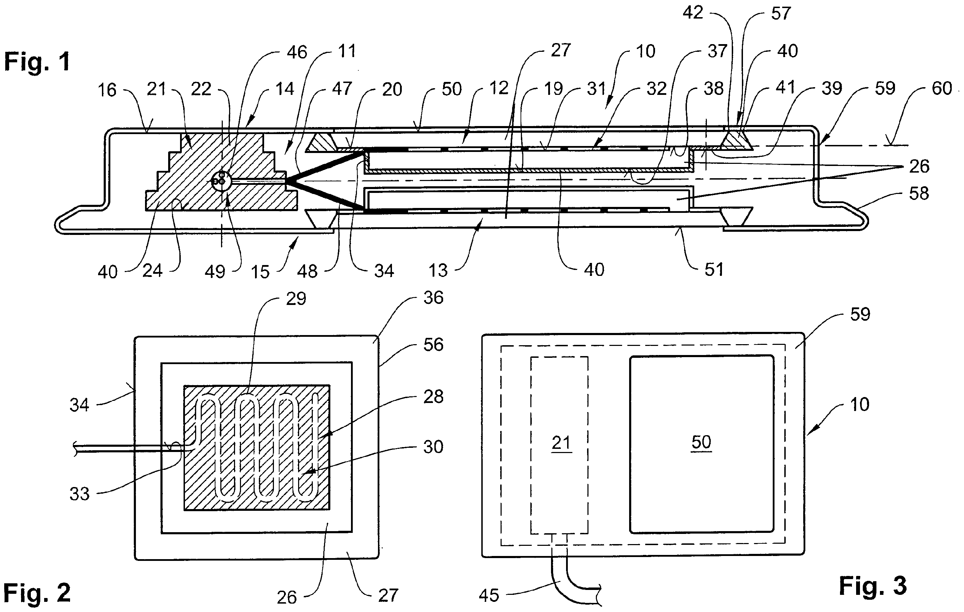

**Fig. 1** zeigt in einer Querschnittsdarstellung eine Ausführungsform eines Sensormoduls 10 mit einer Trägerplatte 11, die bei dem hier dargestellten Ausführungsbeispiel aus einem hochdichten Polyethylen (HDPE) gefertigt ist und zwei in die Trägerplatte 11 eingesetzten Elektroden 12, 13 aufweist.

Das Sensormodul 10 eignet sich insbesondere durch Einschweißen der Trägerplatten zur Integration in Foliendichtsysteme und damit zur Ausbildung einer permanenten Monitoring-Sensorik.

Die in den **Fig. 1** und **4** bis 7 dargestellte Trägerplatte 11 weist an zwei einander gegenüberliegenden Seiten 14, 15 in eine Trägerplattenoberfläche 16 eingebrachte Elektrodenaufnahmen 17, 18 auf, die bevorzugt durch eine Fräsbearbeitung der Trägerplattenoberfläche 16 erzeugt werden und einen jeweils an einem Aufnahmeboden 19 ausgebildeten Auflagerand 20 aufweisen, der im vorliegenden Fall rahmenartig ausgebildet ist. Der Auflagerand 20 ist gegenüber der Trägerplattenoberfläche 16 zurückversetzt, sodass sich insgesamt der in **Fig. 1****,** **4, 5** und **7** dargestellte abgestufte Querschnitt der Elektrodenaufnahmen 17, 18 ergibt. Neben den beiden hier vorzugsweise durch Fräsung eingebrachten Elektrodenaufnahmen 17, 18 weist die Trägerplatte 11 eine insbesondere in den **Fig. 1****,** **4** und **5** dargestellte Kabelaufnahme 21 auf, die vorzugsweise ebenfalls durch Einfräsung erzeugt wird und bei dem dargestellten Ausführungsbeispiel einen Stufenquerschnitt 22 aufweist, der aus übereinander und ineinander übergehend angeordneten, hier quaderförmig ausgebildeten Hohlräumen zusammengesetzt ist, deren Breite b ausgehend von einem Aufnahmeboden 52 sich zur Trägerplattenoberfläche 16 hin reduziert, derart, dass in der Trägerplattenoberfläche 16 eine Aufnahmeöffnung 23 ausgebildet ist.

Weiterhin weist die Trägerplatte 11 zwei jeweils die Elektrodenaufnahmen 17, 18 mit der Kabelaufnahme 21 verbindende Kabeldurchführungen 24, 25 auf, die sich von einer Innenkante des Auflagerands 20 der Elektrodenaufnahmen 17, 18 geneigt zu einer Verbindungsebene 53 (**Fig**. **5**) in der Kabelaufnahme 21 erstrecken.

Wie sich aus einer Zusammenschau der **Fig. 1** und **3** ergibt, weisen die Elektroden 12, 13 jeweils eine innere Kontaktplatte 26 und eine äußere Kontaktplatte 27 auf, zwischen denen ein Sensorelement 28 angeordnet ist, das im vorliegenden Fall einen vorzugsweise mit Gold beschichteten Sensordraht 29 aufweist, der einen Kontaktmäander 30 ausbildet, welcher in einer Verbindungsebene 60 zwischen den Kontaktplatten 26, 27 auf einer Kontaktoberfläche 31 der inneren Kontaktplatte 26 in einer Aufnahmekammer 32 angeordnet ist. Ebenfalls in der Kontaktoberfläche 31 der inneren Kontaktplatte 26 befindet sich eine Sensordrahtdurchführung 33, die die Aufnahmekammer 32 mit einem Außenrand 34 der inneren Kontaktplatte 26 verbindet. Bei der in den **Fig. 1** und **3** dargestellten Relativanordnung der inneren Kontaktplatte 26 und der die innere Kontaktplatte 26 mit einem Randüberstand 56 überragenden äußeren Kontaktplatte 27 ergibt sich für den Kontaktmäander 30 des Sensordrahts 29 ein bis auf die Sensordrahtdurchführung 33 abgeschlossener Aufnahmeraum, aus dem ein Anschlussende 55 des Sensordrahts 29 herausgeführt ist. Zur Erzielung einer maximierten Kontaktoberfläche zwischen dem Kontaktmäander 30 und den Kontaktplatten 26, 27 ist die Aufnahmekammer 32 mit einem Kohlenstoffpulver 35 verfüllt.

Wie insbesondere aus **Fig. 1** ersichtlich, sind die Elektroden 12, 13 so in den Elektrodenaufnahmen 17, 18 angeordnet, dass die äußeren Kontaktplatten 27 zumindest in Kontaktplatteneckbereichen 36 (**Fig. 3**) auf dem Auflagerand 20 aufliegen. Vorzugsweise wird bei dem in **Fig. 1** dargestellten Ausführungsbeispiel ein mechanischer Verbund zwischen der inneren Kontaktplatte 26 und der äußeren Kontaktplatte 27 gleichzeitig mit einem mechanischen Verbund zwischen den Elektroden 12, 13 und der Trägerplatte 11 hergestellt. Hierzu ist sowohl zwischen einer Unterseite 37 der inneren Kontaktplatte 26 und dem Aufnahmeboden 19 als auch zwischen einem an einer Unterseite 38 des Randüberstands 56 der äußeren Kontaktplatte 27 ausgebildeten Auflagerand 39 und dem Auflagerand 20 der Elektrodenaufnahmen 17, 18 ein Kleber aus einem vernetzenden Kunststoffmaterial 40, insbesondere ein PU-Kleber, aufgebracht, der nach Aushärtung einen festen, stabilen mechanischen Verbund zwischen den Elektroden 12, 13 und der Trägerplatte 11 bewirkt. Darüber hinaus ist, wie in **Fig. 1** dargestellt, ein zwischen einem in der Trägerplattenoberfläche 16 ausgebildeter Öffnungsrand 41 und einem Außenrand 42 der äußeren Kontaktplatten 27 ausgebildeter Randspalt 43 ebenfalls mit dem Kunststoffmaterial 40 verfüllt, sodass an der Trägerplattenoberfläche 16 eine vollständige Abdichtung zwischen den Elektroden 12, 13 und der Trägerplattenoberfläche 16 erzeugt ist.

Zur Erhöhung der Stabilität des über das Kunststoffmaterial 40 erzeugten mechanischen Verbunds zwischen der Trägerplatte 11 und den Elektroden 12, 13 ist bei dem in **Fig. 1** dargestellten Ausführungsbeispiel der Randspalt 43 mit einem schwalbenschwanzartig ausgeführten, sich zur Trägerplattenoberfläche 16 hin verjüngenden Spaltquerschnitt 44 versehen.

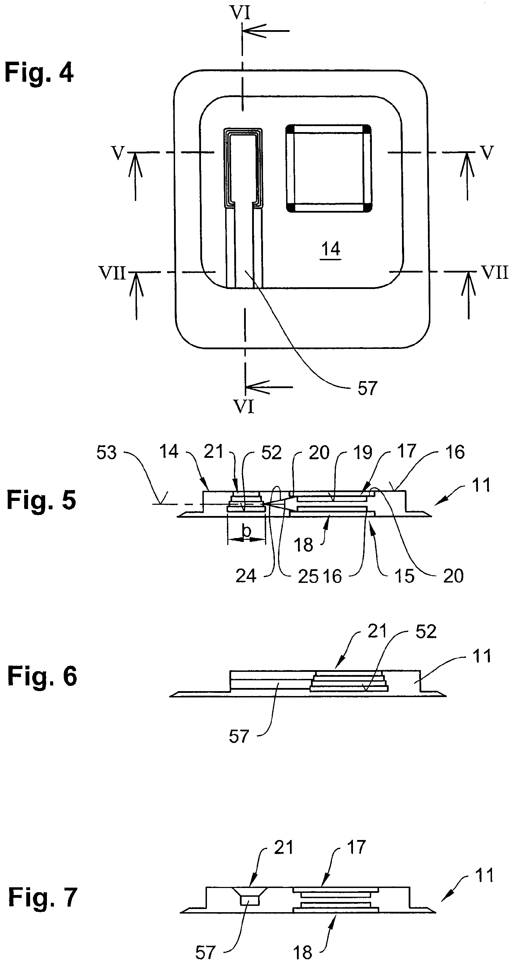

Wie aus einer Zusammenschau der **Fig. 1** und **2** deutlich wird, ist die Trägerplatte 11 mit einer Kabeldurchführung 57 (siehe auch **Fig. 4** und **6**) versehen, die in die Kabelaufnahme 21 einmündet und zur Durchführung eines Anschlusskabels 45 des Sensormoduls 10 dient, das an seinem Anschlussende 46 (**Fig. 1**) mit dem Sensordrahtenden 47, 48 verbunden ist. Zur Zugentlastung und gleichzeitig versiegelnden Aufnahme einer zwischen den Sensordrahtenden 47, 48 und dem Anschlussende 46 des Anschlusskabels 45 ausgeführten Kabelverbindung 49 ist die Kabelaufnahme 21 mit dem Kunststoffmaterial 40 verfüllt.

Um mit Ausnahme der frei zugänglich bleibenden Kontaktflächen 50, 51 der äußeren Kontaktplatten 27 der Elektroden 12, 13 eine durchgängig aus PE-Material gebildete Gehäusung 58 auszubilden, ist die Trägerplatte 11 auf ihrer Trägerplattenoberfläche 16 mit einer Folie 59 abgedeckt bzw. beschichtet, sodass auch die mit dem Kunststoffmaterial 40 verfüllten Öffnungen der Trägerplatte 11, also die Aufnahmeöffnung 23 der Kabelaufnahme 21 und der Randspalt 43, mit der Folie abgedeckt.

## Patentansprüche

1. Sensormodul (10) zur Messung einer elektrischen Größe mit zumindest einer Elektrode (13) die ein zwischen einer inneren und einer äußeren Kontaktplatte (26, 27) aus einem elektrisch leitenden Kohlenstoffmaterial angeordnetes Sensorelement (28) aufweist, das mit einem Anschlußkabel (45) zum Anschluss an eine elektrische Messeinrichtung versehen ist,
**dadurch gekennzeichnet,**
**dass** die Elektrode (13) in einem Gehäuse (58) aus einem elektrisch isolierenden Material angeordnet ist, derart, dass die äußere Kontaktplatte (27) der Elektrode (13) in einer Außenfläche des Gehäuses angeordnet ist.

2. Sensormodul nach Anspruch 1,
**dadurch gekennzeichnet,**
**dass** das Sensorelement (28) in das Kohlenstoffmaterial einer Kontaktplatte (26, 27) eingebettet aufgenommen ist.

3. Sensormodul nach Anspruch 1 oder 2,
**dadurch gekennzeichnet,**
**dass** die Kontaktplatten (26, 27) zur Anordnung der Elektrode (12, 13) eine in einer Verbindungsebene (60) der Kontaktplatten ausgebildete Aufnahmekammer (32) aufweisen.

4. Sensormodul nach Anspruch 3,
**dadurch gekennzeichnet,**
**dass** die Aufnahmekammer (32) zur Einbettung des Sensorelements (28) mit einer elektrisch leitfähigen Füllmasse verfüllt ist.

5. Sensormodul nach Anspruch 4,
**dadurch gekennzeichnet,**
**dass** als leitfähige Füllmasse ein kohlenstoffhaltiges Pulver (35) verwendet wird.

6. Sensormodul nach einem der Ansprüche 3 bis 5,
**dadurch gekennzeichnet,**
**dass** die Aufnahmekammer (32) durch eine der Struktur des Sensorelements (28) angepasste Ausnehmung in zumindest einer Kontaktoberfläche (31) der Kontaktplatten (26, 27) ausgebildet ist.

7. Sensormodul nach Anspruch 6,
**dadurch gekennzeichnet,**
**dass** die Aufnahmekammer (32) durch eine Einfräsung in der Kontaktoberfläche (31) einer Kontaktplatte (26, 27) ausgebildet ist.

8. Sensormodul nach einem der vorangehenden Ansprüche,
**dadurch gekennzeichnet,**
**dass** die Elektrode (12, 13) auf einer Seite (14, 15) einer Trägerplatte (11) des Gehäuses (58) angeordnet ist, derart, dass die Kontaktplatten (26,27) der Elektrode in einer Elektrodenaufnahme (17, 18) in einer Trägerplattenoberfläche (16) angeordnet sind.

9. Sensormodul nach Anspruch 8,
**dadurch gekennzeichnet,**
**dass** die Elektrodenaufnahme (17, 18) mit einem an einem Aufnahmeboden (19) ausgebildeten Auflagerand (20) versehen ist, derart, dass die innere Kontaktplatte (26) auf dem Aufnahmeboden und die äußere Kontaktplatte (27) auf dem Auflagerand angeordnet ist.

10. Sensormodul nach Anspruch 8,
**dadurch gekennzeichnet,**
**dass** zur Fixierung der Kontaktplatten (26, 27) und Abdichtung der Elektrode (12,13) in der Elektrodenaufnahme (17, 18) der Trägerplatte (11) zwischen der äußeren Kontaktplatte (27) und einem Öffnungsrand (41) der Elektrodenaufnahme in der Trägerplattenoberfläche (16) gebildete Randspalten (43) mit einem nicht leitenden, aushärtenden Kunststoffmaterial (40) verfüllt sind.

11. Sensormodul nach einem der vorangehenden Ansprüche,
**dadurch gekennzeichnet,**
**dass** zur Messung einer elektrischen Potenzialdifferenz bzw. eines elektrischen Widerstands zwei Elektroden (12, 13) in dem Gehäuse (58) elektrisch voneinander isoliert angeordnet sind, die über ein Anschlusskabel (45) an eine elektrische Messeinrichtung anschließbar sind, wobei die Sensorelemente (28) der Elektroden jeweils mit dem Anschlusskabel verbunden sind, und jeweils eine äußere Kontaktplatte (27) jeder Elektrode in einer Außenfläche des Gehäuses angeordnet ist.

12. Sensormodul nach Anspruch 11,
**dadurch gekennzeichnet,**
**dass** die Elektroden (12, 13) auf zwei einander gegenüberliegenden Seiten (14, 15) der Trägerplatte (11) des Gehäuses (58) angeordnet sind, derart, dass jeweils die Kontaktplatten (26,27) einer Elektrode in einer Elektrodenaufnahme (17, 18) in einer Trägerplattenoberfläche (16) angeordnet sind.

13. Sensormodul nach Anspruch 12,
**dadurch gekennzeichnet,**
**dass** die Trägerplatte (11) benachbart den einander gegenüberliegend angeordneten Elektrodenaufnahmen (17, 18) in der Trägerplattenoberfläche (16) eine Kabelaufnahme (21) zur Aufnahme einer zwischen einem Anschlussende (46) des Anschlusskabels (45) und Sensordrahtenden (47, 48) der Sensorelemente (28) hergestellten Kabelverbindung (49) aufweist, wobei zur Herstellung der Verbindung des Anschlusskabels mit den Sensordrahtenden die Elektrodenaufnahmen über Kabeldurchführungen (24, 25) mit der Kabelaufnahme (21) verbunden sind.

14. Sensormodul nach Anspruch 13,
**dadurch gekennzeichnet,**
**dass** zur Fixierung des Anschlusskabels (45) in der Kabelaufnahme (21) die Kabelaufnahme mit einem nicht leitenden, aushärtenden Kunststoffmaterial (40) verfüllt ist.

15. Sensormodul nach Anspruch 13 oder 14,
**dadurch gekennzeichnet,**
**dass** die Trägerplatte (11) zumindest auf der die Kabelaufnahme (41) aufweisenden Oberfläche eine Beschichtung mit einer Kunststofffolie (59) aufweist.

## Claims

1. Sensor module (10) for measuring an electric variable comprising at least one electrode(13), which has a sensor element (28), which is arranged between an inner and an outer contact plate (26, 27) made of an electrically conductive carbon material and which is provided with a connecting cable (45) for being connected to an electrical measuring device,
**characterized in that**
the electrode (13) is arranged in a housing (58) made of an electrically insulating material in such a manner that the outer contact plate (27) of the electrode (13) is arranged in an outer face of the housing.

2. The sensor module according to claim 1,
**characterized in that**
the sensor element (28) is received so as to be embedded in the carbon material of a contact plate (26, 27).

3. The sensor module according to claim 1 or 2,
**characterized in that**
the contact plates (26, 27) for arranging the electrode (12, 13) have a receiving chamber (32), which is formed in a coupling plane (60) of the contact plates.

4. The sensor module according to claim 3,
**characterized in that**
the receiving chamber (32) for embedding the sensor element (28) is filled with an electrically conductive filling compound.

5. The sensor module according to claim 4,
**characterized in that**
a powder (35) containing carbon is used as the conductive filling compound.

6. The sensor module according to any one of the claims 3 to 5,
**characterized in that**
the receiving chamber (32) is formed in at least one contact surface (31) of the contact plates (26, 27) by a recess, which is adapted to the structure of the sensor element (28).

7. The sensor module according to claim 6,
**characterized in that**
the receiving chamber (32) is formed by a milled indentation in the contact surface (31) of a contact plate (26, 27).

8. The sensor module according to any one of the preceding claims,
**characterized in that**
the electrode (12, 13) is arranged on a side (14, 15) of a support plate (11) of the housing (58) in such a manner that the contact plates (26,27) of the electrode are arranged in an electrode reception (17, 18) in a support plate surface (16).

9. The sensor module according to claim 8,
**characterized in that**
the electrode reception (17, 18) is provided with a bearing edge (20), which is formed at a receiving bottom (19), in such a manner that the inner contact plate (26) is arranged on the receiving bottom and the outer contact plate (27) is arranged on the bearing edge.

10. The sensor module according to claim 8,
**characterized in that**
for fixing the contact plates (26, 27) and sealing the electrode (12,13) in the electrode reception (17, 18) of the support plate (11), edge gaps (43), which are formed between the outer contact plate (27) and an opening edge (41) of the electrode reception in the support plate surface (16), are filled with a nonconductive curing plastic material (40).

11. The sensor module according to any one of the preceding claims,
**characterized in that**
for measuring an electrical difference in potential or an electrical resistance, two electrodes (12, 13) are arranged in the housing (58) so as to be electrically insulated with respect to each other, said electrodes being able to be connected to an electrical measuring device by means of a connecting cable (45), the sensor elements (28) of the electrodes each being coupled to the connecting cable, and one outer contact plate (27) of each electrode, respectively, being arranged in an outer face of the housing.

12. The sensor module according to claim 11,
**characterized in that**
the electrodes (12, 13) are arranged on two opposite sides (14, 15) of the support plate (11) of the housing (58) in such a manner that the contact plates (26,27) of an electrode are each arranged in an electrode reception (17, 18) in a support plate surface (16).

13. The sensor module according to claim 12,
**characterized in that**
the support plate (11), which is arranged next to the electrode receptions (17, 18), which are arranged opposite from each other, has a cable reception (21) for receiving a cable coupling (49) in the support plate surface (16), said cable coupling (49) being provided between a connecting end (46) of the connecting cable (45) and sensor wire ends (47, 48) of the sensor elements (28), said electrode receptions being coupled to the cable reception (21) via cable channels (24, 25) in order to provide the coupling of the connecting cable to the sensor wire ends.

14. The sensor module according to claim 13,
**characterized in that**
the cable reception is filled with a nonconductive curing plastic material (40) in order to fix the connecting cable (45) in the cable reception (21).

15. The sensor module according to claim 13 or 14,
**characterized in that**
the support plate (11) has a coating comprising a plastic foil (59) on at least the surface that has the cable reception (41).

## Revendications

1. Module capteur (10) pour mesurer une grandeur électrique, comprenant au moins un électrode(13), qui a un élément capteur (28), qui est disposé entre une plaque de contact (26) intérieure et une plaque de contact (27) extérieure fait en matériau carboné électriquement conducteur et qui est pourvu d'un câble de raccordement (45) pour être relié à un dispositif électrique de mesure,
**caractérisé en ce que**
l'électrode (13) est disposé dans un boitier (58) fait en matériau électriquement isolant de telle manière que la plaque de contact (27) extérieure de l'électrode (13) est disposée dans une surface extérieure du boitier.

2. Module capteur selon la revendication 1,
**caractérisé en ce que**
l'élément capteur (28) est reçu de façon à être incorporé dans le matériau carboné d'une plaque de contact (26, 27).

3. Module capteur selon la revendication 1 ou 2,
**caractérisé en ce que**
les plaques de contact (26, 27) pour disposer l'électrode (12, 13) ont une chambre de réception (32) formée dans un plan de contact (60) des plaques de contact.

4. Module capteur selon la revendication 3,
**caractérisé en ce que**
la chambre de réception (32) pour incorporer l'élément capteur (28) est remplie avec une masse de remplissage électriquement conductrice.

5. Module capteur selon la revendication 4,
**caractérisé en ce qu'**
une poudre (35) carburée est utilisée en tant que masse de remplissage conductrice.

6. Module capteur selon l'une quelconque des revendications 3 à 5,
**caractérisé en ce que**
la chambre de réception (32) est formée dans au moins une surface de contact (31) des plaques de contact (26, 27) par un évidement, qui est adapté à la structure de l'élément capteur (28).

7. Module capteur selon la revendication 6,
**caractérisé en ce que**
la chambre de réception (32) est formée par une gravure fraisée dans la surface de contact (31) d'une plaque de contact (26, 27).

8. Module capteur selon l'une quelconque des revendication précédentes,
**caractérisé en ce que**
l'électrode (12, 13) est disposé sur un côté (14, 15) d'une plaque de support (11) du boitier (58) de telle manière que les plaques de contact (26,27) de l'électrode sont disposées dans une réception de électrode (17, 18) dans une surface de plaque de support (16).

9. Module capteur selon la revendication 8,
**caractérisé en ce que**
la réception d'électrode (17, 18) est pourvu à un bord d'appui (20), qui est formé à un fond de réception (19), de telle manière que la plaque de contact (26) intérieure est disposée sur le fond de réception et la plaque de contact (27) extérieure est disposée sur le bord d'appui.

10. Module capteur selon la revendication 8,
**caractérisé en ce que**
pour fixer les plaques de contact (26, 27) et étancher l'électrode (12,13) dans la réception d'électrode (17, 18) de la plaque de support (11), des bords marginaux (43), qui sont formés entre la plaque de contact (27) extérieure et un bord d'ouverture (41) de la réception d'électrode dans la surface de plaque de support (16), sont remplis avec un matériau plastique (40) isolant durcissant.

11. Module capteur selon l'une quelconque des revendication précédentes,
**caractérisé en ce que**
pour mesurer une différence de potentiel électrique ou une résistance électrique, deux électrodes (12, 13) sont disposés dans le boitier (58) de façon à être électriquement isolés l'un de l'autre, lesdits électrodes pouvant être reliés à un dispositif électrique de mesure par un câble de raccordement (45), les éléments capteur (28) des électrodes étant reliés chacun au câble de raccordement et une plaque de contact (27) extérieure de chaque électrode étant disposée dans une surface extérieure du boitier.

12. Module capteur selon la revendication 11,
**caractérisé en ce que**
les électrodes (12, 13) sont disposés sur deux côtés (14, 15) opposés de la plaque de support (11) du boitier (58) de telle manière que les plaques de contact (26,27) d'un électrode sont disposés chacun dans une réception d'électrode (17, 18) dans une surface de plaque de support (16).

13. Module capteur selon la revendication 12,
**caractérisé en ce que**
la plaque de support (11), adjacente aux réceptions d'électrode (17, 18) opposées l'unes aux autres, a un câble de réception (21) pour recevoir une reliaison de câble (49) dans la surface de plaque de support (16), ladite reliaison de câble (49) étant pourvue entre une extrémité de reliaison (46) du câble de raccordement (45) et des extrémités de fil capteur (47, 48) des éléments capteur (28), les réceptions d'électrode étant reliées au câble de réception (21) par des passages de câble (24, 25) pour pourvoir la reliaison du câble de raccordement aux extrémités de fil capteur.

14. Module capteur selon la revendication 13,
**caractérisé en ce que**
la réception de câble est remplie avec un matériau plastique (40) isolant durcissant pour fixer le câble de raccordement (45) dans la réception de câble (21).

15. Module capteur selon la revendication 13 ou 14,
**caractérisé en ce que**
la plaque de support (11) a un revêtement qui comprend une feuille plastique (59) au moins sur la surface qui a la réception de câble (41).
